(19) Europäisches Patentamt
European Patent Office
Office européen des brevets

(11) Veröffentlichungsnummer : **0 315 783 B1**

# (12) EUROPÄISCHE PATENTSCHRIFT

(45) Veröffentlichungstag der Patentschrift : **12.06.91 Patentblatt 91/24**

(51) Int. Cl.[5] : **C07C 17/00,** C07C 23/06, C07C 23/08, C09K 3/30, C09K 5/00

(21) Anmeldenummer : **88116781.1**

(22) Anmeldetag : **10.10.88**

## (54) Verfahren zur Herstellung von fluorierten C4- bis C6-Kohlenwasserstoffen und neue cyclische fluorierte Kohlenwasserstoffe, sowie die Verwendung von fluorierten C4- bis C6-Kohlenwasserstoffen als Treibgas und Arbeitsflüssigkeit für Wärmepumpensysteme.

(30) Priorität : **20.10.87 DE 3735467**

(43) Veröffentlichungstag der Anmeldung : **17.05.89 Patentblatt 89/20**

(45) Bekanntmachung des Hinweises auf die Patenterteilung : **12.06.91 Patentblatt 91/24**

(84) Benannte Vertragsstaaten : **BE CH DE ES FR GB IT LI NL**

(56) Entgegenhaltungen : **Keine.**

(73) Patentinhaber : **BAYER AG**
**W-5090 Leverkusen 1 Bayerwerk (DE)**

(72) Erfinder : **Bielefeldt, Dietmar, Dr.**
**Beuthener Strasse 13**
**W-4030 Ratingen 6 (DE)**
Erfinder : **Marhold, Albrecht, Dr.**
**Carl-Duisberg-Strasse 329**
**W-5090 Leverkusen 1 (DE)**
Erfinder : **Negele, Michael, Dr.**
**Wolfskaul 6**
**W-5000 Köln 80 (DE)**

EP 0 315 783 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Herstellung von linearen und cyclischen, fluorierten $C_4$– bis $C_6$-Kohlenwasserstoffen durch katalytische Hydrierung entsprechender Halogenolefine, neue cyclische fluorierte Kohlenwasserstoffe und die Verwendung von fluorierten $C_4$- bis $C_6$-Kohlenwasserstoffen als Treibgas und Arbeitsflüssigkeit für Wärmepumpensysteme.

Es sind bereits Verfahren zur Herstellung spezieller fluorierter $C_4$-Kohlenwasserstoffe bekannt.

So kann man 1,1,1,4,4,4-Hexafluorbutan herstellen, indem man Bernsteinsäure mit Schwefeltetrafluorid umsetzt (siehe W. Dmowski et al., PL-Erfinderschein 87 481). Dieses Verfahren ist nicht befriedigend, da für es toxisches und nur auf unwirtschaftliche Weise zugängliches Schwefeltetrafluorid benötigt wird. Man kann 1,1,1,4,4,4-Hexafluorbutan auch herstellen, indem man 1,1,1,4,4,4-Hexafluor-2-chlor-buten-2 oder 1,1,1,4,4,4-Hexafluor-2,3-dichlor-buten-2 katalytisch in Gegenwart von Palladium auf Aluminiumoxid hydriert (siehe Y, Huang et al., Youji Huaxve 2, 125 (1984). Nachteilig hierbei ist, daß das Produkt stets in Form von Gemischen mit chlorhaltigen Verbindungen anfällt. Nach neueren Untersuchungen [siehe J.F.D. Mills, Cell. Polym. 5, 343 (1986) und F.S. Rowland et al., Nature 239, 8 (1974)] ist jedoch gerade der Chlorgehalt von konventionellen Treibgasen schädlich für die Ozonschicht der Erdatmosphäre. Schließlich kann man 1,1,1,4,4,4-Hexafluorbutan auch durch Hydrierung von 1,1,1,4,4,4-Hexafluorbuten-2 herstellen [siehe R. N. Haszeldine, J. Chem. Soc., 2504 (1952)]. Nachteilig hierbei ist der Einsatz eines teuren und schwierig zugänglichen Ausgangsprodukts (siehe R.N. Haszeldine a. a. O.).

1,1,2,2-Tetrafluorcyclobutan kann man durch Umsetzung von Tetrafluorethylen mit Ethylen in einer Zwei+Zwei-Addition herstellen (siehe D. Coffman et al., JACS 71, 490 (1949)). Die Ausbeute bei dieser Reaktion ist jedoch nicht befriedigend.

Es wurde nun ein Verfahren zur Herstellung von fluorierten $C_4$- bis $C_6$-Kohlenwasserstoffen der Formel (I)

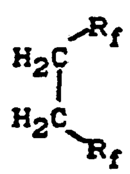

(I),

in der

$R_f$ für $CF_3$ steht oder beide $R_f$ gemeinsam für $-CF_2-CF_2-$, $-CF_2-CF_2-CF_2-$ oder $-CH(CF_3)-CH(CF_3)-$ stehen, gefunden, das dadurch gekennzeichnet ist, daß man Verbindungen der Formel (II)

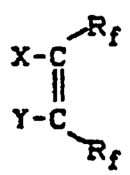

(II),

in der

X für Wasserstoff, Fluor, Chlor oder Brom steht
Y für Fluor, Chlor oder Brom steht und
$R_f$ die bei Formel (I) angegebene Bedeutung hat,
katalytisch in Gegenwart einer Base hydriert.

Für das erfindungsgemäße Verfahren geeignete Ausgangsmaterialien der Formel (II) sind beispielsweise 1,1,1,4,4,4-Hexafluor-2-chlor-buten-2, 1,1,1,2,4,4,4-Heptafluorbuten-2, 1,1,1,4,4,4-Hexafluor-2,3-dichlorbuten-2, 3,3,4,4-Tetrafluor-1-chlor-cyclobuten, 3,3,4,4-Tetrafluor-1,2-dichlor-cyclobuten, 3,3,4,4,5,5-Hexafluor-1-chlor-cyclopenten, 3,3,4,4,5,5-Hexafluor-1,2-dichlor-cyclopenten, 3,4-Di-(Trifluormethyl)-1-chlorcyclobuten, 3,4-Di-(Trifluormethyl)-1,2-dichlor-cyclobuten, 1,1,1,4,4,4-Hexafluor-2,3-dibrom-buten-2 und 1,1,1,4,4,4-Hexafluor-2-brom-3-chlorbuten-2.

Man muß nicht zwingend Verbindungen der Formel (II) einsetzen, man kann auch von Vorläufern ausgehen, die intermediär Verbindungen der Formel (II) ergeben. Bei Vorläufern von Verbindungen der Formel (II) kann es sich beispielsweise um Verbindungen der Formel (III) handeln

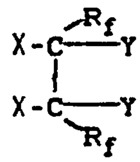 (III),

in der
jedes X und jedes Y unabhängig voneinander und $R_f$ die bei Formel (I) bzw. (II) angegebene Bedeutung haben.

Die Verbindungen der Formel (III) können beispielsweise durch Halogenwasserstoff-Eliminierung, gegebenenfalls mit vorhergehendem Austausch von Halogen gegen Wasserstoff, in Verbindungen der Formel (II) übergehen. Beispiele für Verbindungen der Formel (III) sind 1,1,1,4,4,4-Hexafluor-2,2,3-trichlor-butan, 1,1,1,4,4,4-Hexafluor-2,2,3,3-tetrachlor-butan und 1,1,1,4,4,4-Hexafluor-2,3-dibrom-2-chlor-butan.

Bevorzugt wird in das erfindungsgemäße Verfahren 1,1,1,4,4,4-Hexafluor-2-chlorbuten, 3,3,4,4-Tetrafluor-1,2-dichlor-cyclobuten, 3,3,4,4,5,5,-Hexafluor-1,2-dichlor-cyclopenten oder 1,1,1,2,4,4,4-Heptafluor-buten-2 eingesetzt.

Die Ausgangsprodukte für das erfindungsgemäße Verfahren sind beispielsweise gemäß DE-OS 37 25 213 und/oder H.L. Henne et al., JACS 67, 1235 (1945) und 73, 1103 (1951) auf einfache Weise gut zugänglich.

Als Katalysatoren kommen für das erfindungsgemäße Verfahren vor allem solche in Frage, die Metalle enthalten oder daraus bestehen. Geeignet sind beispielsweise die Metalle der VIII. Nebengruppe des Periodischen Systems der Elemente, insbesondere Palladium, Platin und Nickel. Die Metalle können in elementarer Form oder in Form von Verbindungen (z.B. als Oxide, Hydroxide) eingesetzt werden, sowie in besonders aktivierten Formen, z.B. in Form von Raney-Metallen oder aufgebracht auf Trägermaterialien. Bevorzugt ist Raney-Nickel, Palladium auf Kohle, Aluminiumoxid, Kieselsäure, Bariumsulfat, Calciumcarbonat, Lithium-Aluminium-Spinell, Kieselgel oder Magnesiumoxid.

Es sind auch Katalysatoren verwendbar, die zwei oder mehr Metalle enthalten, beispielsweise Nickel und Eisen. Die Katalysatoren können auch in beliebiger Weise mit Zusatzkomponenten dotiert sein.

Die Menge an Katalysator ist im allgemeinen nicht kritisch. Beispielsweise kann man von 1 bis 100 Gew.-% Katalysator, bezogen auf die eingesetzte Verbindung der Formel (II) verwenden. Die vorstehende Zahlenangabe bezieht sich dabei auf die katalytisch aktiven Bestandteile der Katalysatoren, bei der Verwendung von Trägerkatalysatoren ist also dabei das Gewicht der Trägermaterialien nicht berücksichtigt.

Als Basen kommen für das erfindungsgemäße Verfahren die verschiedensten anorganischen und organischen alkalisch reagierenden Verbindungen in Frage. Als Beispiele seien genannt die Oxide, Hydroxide, Acetate, Carbonate und Hydrogencarbonate von Alkali- und Erdalkalimetallen und tertiäre Amine.

Vorzugsweise kommen Kaliumhydroxid, Natriumhydroxid, Natriumacetat, Triethylamin oder Pyridin zum Einsatz.

Die Basen können in verschiedenen Mengen eingesetzt werden. Beim Einsatz von Verbindungen der Formel (II) mit X = Wasserstoff verwendet man vorzugsweise 0,8 bis 1,2 Äquivalente Base pro Mol Verbindung der Formel (II), beim Einsatz von Verbindungen der Formel (II) mit X = Chlor vorzugsweise 1,8 bis 3 Äquivalente Base pro Mol Verbindung der Formel (II).

Die erfindungsgemäße Hydrierung kann bei unterschiedlichen Drucken und Temperaturen durchgeführt werden. Geeignet sind beispielsweise Drucke im Bereich 1 bis 200 bar und Temperaturen im Bereich 0 bis 200°C. Bevorzugt sind Drucke im Bereich von 1 bis 60 bar und Temperaturen im Bereich von 20 bis 60°C.

Das erfindungsgemäße Verfahren führt man vorzugsweise in Gegenwart eines Lösungsmittels durch. Geeignete Lösungsmittel sind beispielsweise Alkohole, wie Methanol und Ethanol, Ether, wie Tetrahydrofuran und Diglyme, Aromaten, wie Toluol und Essigsäure.

Man kann dieses Verfahren sowohl diskontinuierlich als auch kontinuierlich durchführen. Bei kontinuierlicher Arbeitsweise ordnet man den Katalysator vorzugsweise im Festbett an.

Aufarbeitung des Reaktionsgemisches kann beispielsweise so erfolgen, daß man zunächst gegebenenfalls vorhandene Feststoffe abtrennt und anschließend aus dem Filtrat das Lösungsmittel. Man kann auch so verfahren, daß man das vom Katalysator befreite Reaktionsgemisch auf Eiswasser gibt, die sich bildende organische Phase abtrennt und diese fraktioniert destilliert. Man kann das Reaktionsgemisch auch auf andere Art aufarbeiten.

Das erfindungsgemäße Verfahren hat beispielsweise folgende Vorteile : Es erfordert keine schwierig zugänglichen Ausgangsstoffe und Reagenzien, es liefert reine Produkte in guten Ausbeuten und es stellt einen ökonomischen Weg zu fluorierten, jedoch chlorfreien Kohlenwasserstoffen zur Verfügung.

Die vorliegende Erfindung betrifft weiterhin neue cyclische fluorierte Kohlenwasserstoffe der Formel (Ia)

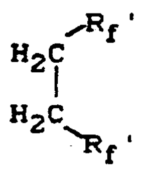

(Ia),

in der
beide $R_f'$ gemeinsam für $-CF_2-CF_2-CF_2-$ oder $-CH(CF_3)-CH(CF_3)-$ stehen.

Im einzelnen handelt es sich dabei um 1,1,2,2,3,3-Hexafluor-cyclopentan und 1,2-Di-(Trifluormethyl)-cyclobutan.

Ein Verfahren zur Herstellung der neuen Verbindungen der Formel (Ia) ist weiter oben, eine technische Verwendungsmöglichkeit weiter unten beschrieben.

Die vorliegende Erfindung betrifft weiterhin die Verwendung von Verbindungen der Formel (I)

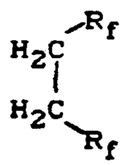

(I),

in der
$R_f$ für $CF_3$ steht oder beide $R_f$ gemeinsam für $-CF_2-CF_2-$, $-CF_2-CF_2-CF_2-$ oder $-CH(CF_3)-CH(CF_3)-$ stehen, als Treibgas.

Bevorzugt werden diese Verbindungen als Treibgas für Sprays verwendet, die für die verschiedensten Zwecke eingesetzt werden können, z.B. als Sprays für kosmetische Zwecke (wie Deodorant-Sprays). Besonders bevorzugt werden diese Verbindungen als Treibgas in Sprays für medizinische Zwecke verwendet z.B. in Sprays für Asthmatiker oder in Flüssig-Pflaster-Sprays.

Für diese Verwendung ist das 1,1,1,4,4,4-Hexafluorbutan besonders bevorzugt.

Sprays, die gemäß der vorliegenden Erfindung Verbindungen der Formel (I) als Treibgas enthalten, zeichnen sich dadurch aus, daß sie inert und nicht brennbar sind, wie die bisher zu diesem Zweck häufig verwendeten fluorierten und chlorierten Kohlenwasserstoffe auch. Darüber hinaus wird jedoch die Ozonschicht der Erdatmosphäre durch Verbindungen der Formel (I) praktisch nicht beeinflußt, weil sie chlorfrei sind.

Die vorliegende Erfindung betrifft auch noch die Verwendung von Verbindungen der Formel (Ib)

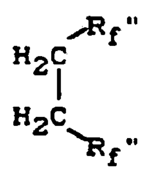

(Ib)

in der
$R_f''$ für $CF_3$ steht oder beide $R_f''$ gemeinsam für $-CF_2-CF_2-CF_2-$ oder $-CH(CF_3)-CH(CF_3)-$ stehen, als Arbeitsflüssigkeit für Wärmepumpensysteme.

Beispiele

Beispiel 1

In einem Edelstahlautoklaven wurden 40 g 1,1,1,4,4,4-Hexafluor-2-chlor-buten-2 in 300 ml Ethanol in Gegenwart von 12 g Kaliumhydroxid und 25 g Raney-Nickel 3 Stunden lang bei 20°C und 1 Stunde bei 100°C mit Wasserstoff hydriert. Der Druck lag dabei im Bereich von 30 bis 40 bar. Danach wurden aus dem Reaktionsgemisch die festen Bestandteile durch Filtration abgetrennt und die verbleibende Flüssigkeit durch Destillation aufgearbeitet. Es wurden 16 g 1,1,1,4,4,4-Hexafluorbutan mit einem Siedepunkt von 25 bis 30°C bei 1013 mbar erhalten. Das Massenspektrum zeigte für $m^+/e$ den Wert 166.

Beispiel 2

199 g (1 Mol) 1,1,1,4,4,4-Hexafluor-2-chlor-buten-2 wurden in 800 ml Diglyme und in Gegenwart von 45 g Natriumhydroxid und 30 g Raney-Nickel im Temperaturbereich von 20 bis 40°C und bei einem Wasserstoffdruck von 20 bis 40 bar hydriert. Nach dem Abfiltrieren der festen Bestandteile wurde das Lösungsmittel mit Wasser extrahiert und die abgetrennte organische Phase durch fraktionierte Destillation gereinigt. Die Ausbeute an 1,1,1,4,4,4-Hexafluorbutan betrug 125 g (75% d.Th.), der Siedepunkt 24 bis 27°C bei 1013 mbar. Das $^{19}F$-NMR-Spektrum zeigte einen peak bei −10,7 ppm (standard : $CF_3CO_2H$).

Beispiel 3

10 g (36 mMol) 1,1,1,4,4,4-Hexafluor-2-brom-3-chlorbuten-2 wurden in 50 ml Tetrahydrofuran in Gegenwart von 3,0 g Natriumhydroxid und 5 g Raney-Nickel im Temperaturbereich von 20 bis 40°C und bei einem Wasserstoffdruck von 20 bis 40 bar hydriert. Die Aufarbeitung erfolgte wie in Beispiel 2 beschrieben. Die Ausbeute betrug 3,5 g 1,1,1,4,4,4-Hexafluorbutan (= 59% d.Th.).

Beispiel 4

40 g (0.2 Mol) 1,1,1,4,4,4-Hexafluor-2-chlor-buten-2 wurden mit 12 g Kaliumhydroxid in 300 ml Ethanol im Druckbereich von 20 bis 40 bar und bei einer Temperatur von 20 bis 100°C in Gegenwart von 25 g Raney-Nickel hydriert. Nach dem Abfiltrieren der festen Bestandteile wurde das Lösungsmittel mit Wasser extrahiert und die abgetrennte organische Phase durch Destillation gereinigt. Es wurden 15,5 g (= 47% d.Th.) 1,1,1,4,4,4-Hexafluorbutan erhalten. Der Siedepunkt betrug 24 bis 27°C bei 1013 mbar.

Beispiel 5

23,5 g (0.1 Mol) 1,1,1,4,4,4-Hexafluor-2,3-dichlor-buten-2 wurden mit 50 ml Tetrahydrofuran, 8,5 g Natriumhydroxid und 3 g eines Katalysators versetzt, der 5 Gew.-% Palladium auf Kohle enthielt. Dieses Gemisch wurde bei Temperaturen zwischen 20 und 40°C mit Wasserstoff bei Drucken im Bereich 20 bis 40 bar hydriert. Die Aufarbeitung des Reaktionsgemisches erfolgte wie in Bei spiel 2 beschrieben. Die Ausbeute betrug 8,0 g (= 75% d.Th.) 1,1,1,4,4,4-Hexafluorbutan.

Beispiel 6

In einem 1.3 1-Edelstahlautoklaven wurden 245 g (1 Mol) 1,2-Dichlor-3,3,4,4,5,5-hexafluorcyclopenten unter Zusatz von 202 g (2 mol) Triethylamin in 200 ml Methanol und in Gegenwart von 20 g Raney-Nickel bei 60-70°C hydriert. Innerhalb von 12 Stunden wurde bei einem Wasserstoffdruck von 40-50 bar die theoretische Menge Wasserstoff aufgenommen. Das Reaktionsgemisch wurde filtriert und die methanolische Lösung mit 400 ml Wasser verdünnt. Die untere organische Phase wurde abgetrennt, mit 100 ml 5%-iger Salzsäure gewaschen und über Natriumsulfat getrocknet. Durch Destillation über eine 1m – Drehbandkolonne wurden 106 g (60% d. Th.) 1,1,2,2,3,3-Hexafluorcyclopentan mit einem Siedepunkt von 87-88°C bei 1013 mbar erhalten. Das Massenspektrum zeigte das Molekülion bei m/e = 178.

$n_D^{20}$ : 1. 309

$^1H$-NMR : = 2,25-2,5 ppm (m, 4H, gg. int. TMS)

$^{19}F$-NMR : = -36.5 ppm (tt, 4F, gg. ext. $CF_3COOH$)
u.-57.9 ppm (m, 2F)

## Ansprüche

1. Verfahren zur Herstellung von fluorierten $C_4$– bis $C_6$-Kohlenwasserstoffen der Formel

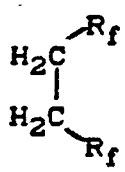

,

in der
$R_f$ für $CF_3$ steht oder beide $R_f$ gemeinsam für $-CF_2-CF_2-$, $-CF_2-CF_2-CF_2-$ oder $-CH(CF_3)-CH(CF_3)-$ stehen, dadurch gekennzeichnet, daß man Verbindungen der Formel

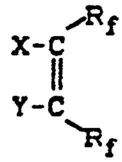

,

in der
X für Wasserstoff, Fluor, Chlor oder Brom
Y für Fluor, Chlor oder Brom steht und
$R_f$ die oben angegebene Bedeutung hat,
katalytisch in Gegenwart einer Base hydriert.

2. Verfahren nach Anspruch 1, dadurch gekennzeichnet, daß man 1,1,1,4,4,4-Hexafluor-2-chlorbuten, 3,3,4,4-Tetrafluor-1,2-dichlor-cyclobuten, 3,3,4,4,5,5-Hexafluor-1,2-dichlor-cyclopenten oder 1,1,1,2,4,4,4-Heptafluorbuten-2 einsetzt.

3. Verfahren nach Anspruch 1, dadurch gekennzeichnet, daß man Vorläufer der Verbindungen der Formel

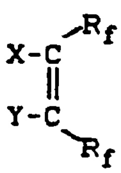

einsetzt, die intermediär solche Verbindungen ergeben.

4. Verfahren nach Ansprüchen 1 bis 3, dadurch gekennzeichnet, daß man als Base Oxide, Hydroxide, Acetate, Carbonate und/oder Hydrogencarbonate von Alkali- und Erdalkalimetallen und/oder tertiäre Amine einsetzt.

5. Verfahren nach Ansprüchen 1 bis 4, dadurch gekennzeichnet, daß man beim Einsatz von Verbindungen mit X = Wasserstoff 0,8 bis 1,2 Äquivalente Base pro Mol Ausgangsverbindung und beim Einsatz von Verbindungen mit X = Chlor 1,8 bis 3 Äquivalente Base pro Mol Ausgangsverbindung einsetzt.

6. Verfahren nach Ansprüchen 1 bis 5, dadurch gekennzeichnet, daß man bei Drucken im Bereich von 1 bis 200 bar und Temperaturen im Bereich von 0 bis 200°C arbeitet.

7. 1,1,2,2,3,3-Hexafluor-cyclopentan und 1,2-Di-(Trifluormethyl)-cyclobutan.

8. Verwendung von Verbindungen der Formel

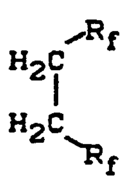

,

in der
$R_f$ für $CF_3$ steht oder beide $R_f$ gemeinsam für $-CF_2-CF_2-$, $-CF_2-CF_2-CF_2-$ oder $-CH(CF_3)-CH(CF_3)-$ stehen,

als Treibgas.

9. Verwendung von Verbindungen der Formel

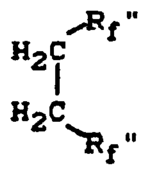
,

in der

$R_f''$ für $CF_3$ steht oder beide $R_f''$ gemeinsam für $-CF_2$-$CF_2$-$CF_2-$ oder $-CH(CF_3)$-$CH(CF_3)-$ stehen, als Arbeitsflüssigkeit für Wärmepumpensysteme.

## Claims

1. A process for the production of fluorinated $C_{4-6}$ hydrocarbons corresponding to the following formula

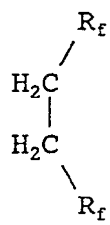

in which

$R_f$ represents $CF_3$ or both $R_f$'s together represent $-CF_2$-$CF_2-$, $-CF_2$-$CF_2$-$CF_2-$ or $-CH$ $(CF_3)$-$CH$ $(CF_3)-$, characterized in that compounds corresponding to the following formula

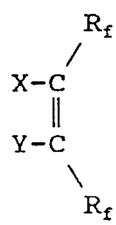

in which

X is hydrogen, fluorine, chlorine or bromine,

Y is fluorine, chlorine or bromine and

$R_f$ is as defined above,

are catalytically hydrogenated in the presence of a base.

2. A process as claimed in claim 1, characterized in that 1,1,1,4,4,4-hexafluoro-2-chlorobutene,3,3,4,4-tetrafluoro-1,2-dichlorocyclobutane, 3,3,4,4,5,5-hexafluoro-1,2-dichlorocyclopentene or 1,1,1,2,4,4,4-heptafluoro-2-butene is used.

3. A process as claimed in claim 1, characterized in that precursors of the compounds

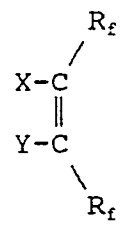

which give such compounds as intermediates are used.

4. A process as claimed in claims 1 to 3, characterized in that oxides, hydroxides, acetates, carbonates and/or hydrogen carbonates of alkali and alkaline earth metals and/or tertiary amines are used as the base.

5. A process as claimed in claims 1 to 4, characterized in that, where compounds in which X = hydrogen are used, 0.8 to 1.2 equivalents base are used per mol starting compound and, where compounds in which X = chlorine are used, 1.8 to 3 equivalents base are used per mol starting compound.

6. A process as claimed in claims 1 to 5, characterized in that it is carried out under pressures of 1 to 200 bar and at temperatures in the range from 0 to 200°C.

7. 1,1,2,2,3,3-hexafluorocyclopentane and 1,2-di-(trifluoromethyl)-cyclobutane.

8. The use of compounds corresponding to the formula

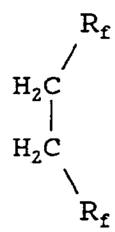

in which
$R_f$ represents $CF_3$, or both $R_f$'s together represent $-CF_2-CF_2-$, $-CF_2-CF_2-CF_2-$ or $-CH(CF_3)-CH(CF_3)-$, as a propellent gas.

9. The use of compounds corresponding to the following formula

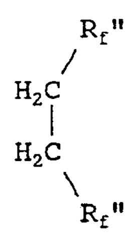

in which
$R_f''$ represents $CF_3$ or both $R_f''$ 's together represent $-CF_2-CF_2-CF_2-$ or $-CH(CF_3)-CH(CF_3)-$, as working fluid for heat pumping systems.

**Revendications**

1. Procédé de fabrication d'hydrocarbures fluorés $C_4$ à $C_6$ de formule

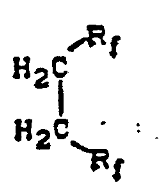
,

dans laquelle
$R_f$ représente $CF_3$ ou les deux $R_f$ représentent ensemble $-CF_2-CF_2$, $-CF_2-CF_2-CF_2-$ ou $-CH(CH_3)-CH\ CH_3)-$, caractérisé en ce qu'on hydrogène catalytiquement des composés de formule

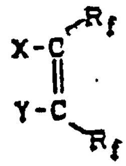,

dans laquelle
X représente de l'hydrogène, du fluor, du chlore ou du brome,
Y représente du fluor, du chlore ou du brome
$R_f$ a la signification susmentionnée
en présence d'une base.

2. Procédé selon la revendication 1, caractérisé en ce qu'on utilise du 1,1,1,4,4,4-hexafluoro-2-chlorobutène, du 3,3,4,4-tétrafluoro-1,2-dichlorocyclobutène, du 3,3,4,4,5,5-hexafluoro-1,2-dichlorocyclopentène ou du 1,1,1,2,4,4,4-heptafluorobutène-2.

3. Procédé selon la revendication 1, caractérisé en ce qu'on utilise des précurseurs des composés de formule

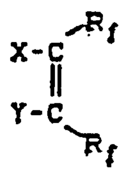,

qui donnent en guise de composés intermédiaires de tels composés.

4. Procédé selon les revendications 1 à 3, caractérisé en ce qu'on utilise comme base des oxydes, des hydroxydes, des acétates, des carbonates et/ou des hydrogénocarbonates de métaux alcalins et alcalino-terreux et/ou des amines tertiaires.

5. Procédé selon les revendications 1 à 4, caractérisé en ce que, en cas d'utilisation de composés avec X = hydrogène, on utilise 0,8 à 1,2 équivalent de base par mole de composé de départ et, en cas d'utilisation de composés avec X = chlore, 1,8 à 3 équivalents de base par mole de composé de départ.

6. Procédé selon les revendications 1 à 5, caractérisé en ce qu'on travaille à des pressions dans l'intervalle de 1 à 200 bar et à des températures dans l'intervalle de 0 à 200°C.

7. 1,1,2,2,3,3-hexafluorocyclopentane et 1,2-di(trifluorométhyl)cyclobutane.

8. Mise en oeuvre de composés de formule

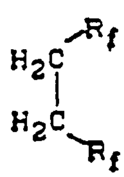,

dans laquelle
$R_f$ représente $CF_3$ ou les deux $R_f$ représentent ensemble $-CF_2-CF_2-$, $-CF_2-CF_2-CF_2-$ ou $-CH\ (CF_3)-CH\ (CF_3)-$ comme gaz propulseur.

9. Mise en oeuvre de composés de formule

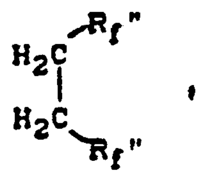

dans laquelle
$R_f''$ représente $CF_3$ ou les deux $R_f''$ représentent ensemble $-CF_2-CF_2-CF_2-$ ou $-CH(CF_3)-CH(CF_3)-$ comme liquide de travail pour des systèmes de pompe à chaleur.